# EUROPEAN PATENT APPLICATION

(11) **EP 1 051 989 A2**
(43) Date of publication of application: **15.11.2000**
(21) Application number: 00109485.3
(22) Date of filing: 04.05.2000
(51) Int. Cl.: A61M 25/00

(54) **Ureteral catheter stent**

(30) Priority: 07.05.1999 IT TO990078
(71) Applicant: Karbix Establishment, 9490 Vaduz (LI)
(72) Inventor: Ugo, Ferrando, 10128 Torino (IT)
(74) Representative: Lotti, Giorgio

(57) **Abstract**

A ureteral catheter stent (1) presenting a main duct (3) extending along a respective longitudinal axis (A), and also presenting two end portions (4) of the duct (3), which are substantially semi-circular, and an intermediate linking portion (13) between the said two portions (4) presenting a diameter (⌀ᵢ) which is variable along the longitudinal axis (A) itself.

## Description

The present invention refers to a ureteral catheter stent.

In the field of urology, ureteral catheter stents of a well-known kind are installed inside a ureter in order to maintain urinary drainage and such ureteral catheter stents comprise: main duct with a constant diameter; a number of drainage holes obtained along the duct itself and arranged in two longitudinally diametrically opposed rows; and two opposing curved end portions.

Ureteral catheter stents of the kind described above are normally used inn all the applications which involve the use of the ureter to prevent any eventual occlusions in the ureter itself, but the shape of the main duct limits their use to a relatively great extent, in that the substantial degree of contact which exists between the main duct and the internal surface of the ureter is not strictly necessary and can also, in fact, frequently be counter-productive.

The aim of the present invention is to realise a ureteral catheter stent, which is able to resolve the disadvantages described above in a simple and cost-effective manner.

According to the present invention, there will be realised a ureteral catheter stent comprising a main duct extending along a respective longitudinal axis, and two curved end portions of the duct which are substantially semi-circular, and which are characterised by the fact that the said main duct comprises an intermediate linking portion between the said two curved end portions presenting a diameter which is variable along the said longitudinal axis.

The invention will no be described with reference to the attached drawings, which illustrate a non-limiting embodiment of the invention, in which:
- FIG. 1: is a side view of a preferred form of embodiment of a ureteral catheter stent according to the present invention; and
- FIG. 2: is a front view of the ureteral catheter stent shown in FIG. 1.

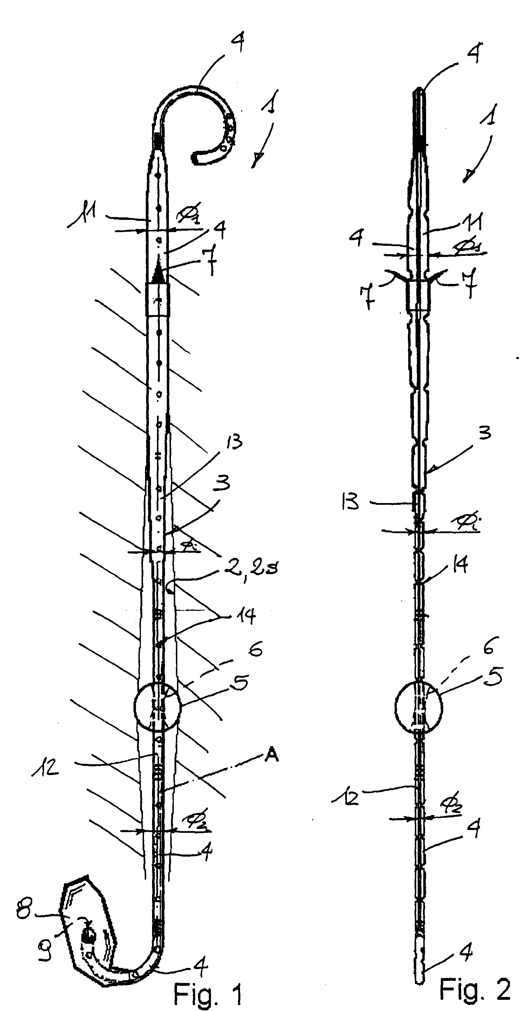

With reference initially to FIG. 1, With reference too the attached drawings, the number 1 refers to, in its entirety, a ureteral catheter stent for the drainage of fluids suitable for being arranged, preferably but not necessarily, inside a ureter 2.

The ureteral catheter stent 1 comprises a main duct 3, which presents a longitudinal axis A and two curved end portions 4 arranged at opposite ends of the duct 3 itself, and being substantially hook-shaped. The ureteral catheter stent 1 is suitable for being inserted along the ureter 2 in such a way that one hooked portion 4 or proximal portion 4a is arranged in the upper excretory tract (not illustrated) of the ureter 2 itself, and in such a way that the other curved end portion 4 or distal portion 4b is arranged inside a container (not illustrated) which can be defined, according to each specific case, by a bladder or a collecting bag.

The ureteral catheter stent 1 also comprises two pairs of anchoring flaps 7 arranged along the duct 3, outside the duct 3 itself, in order to prevent any possible migration of the ureteral catheter stent 1 itself along the ureter 2, and anti-reflux-urine valve 8 arranged in correspondence to the distal portion 4b.

In the case illustrated in the attached drawings, the distal portion 4b presents an opening 9 arranged in correspondence to the end of the portion 4b itself, while the proximal portion 4a is a closed portion. As an alternative, the proximal portion 4a can also present a respective opening identical to the opening 9.

The main duct 3 comprises two tubular portions 11 and 12 which are connected to the relative portions 4, an intermediate linking portion 13 interposed between the two tubular portions 8, and a number of holes 14 for lubrication, which are distributed along the duct 3 itself in two diametrically opposite rows.

The tubular portion 11 is interposed between the proximal portion 4a and the intermediate portion 13, and it presents a determined diameter ⌀₁ which is constant along the entire axial length of the tubular portion 11 itself, while the tubular portion 12 is interposed between the distal portion 4b and the intermediate portion 13, and it presents a determined diameter ⌀₂ which is constant along the entire axial length of the tubular portion 12, and is less than the diameter ⌀₁. The intermediate portion 13 presents, instead, a variable diameter ⌀₁, with a maximum equal to the diameter ⌀₁ and a minimum equal to the diameter ⌀₂.

According to a form of embodiment of the ureteral catheter sent 1 which is not illustrated, but which may be deduced from the illustrations shown in the attached drawings, the tubular portion 11 presents a diameter ⌀₁, and less than the diameter ⌀₂ of the tubular portion 12, and furthermore the holes 14 are partially distributed along the duct 3; or they could be present either along the tubular portion 11 only, or along the tubular portion 12 only, or along the intermediate portion 13 only, or they could be present at the same time along only two of any of the portions listed above.

It is obvious from the description that has been given above that the ureteral catheter stent 1 which has just been described can be used in situations where it would be superfluous to have a duct of the same diameter along its entire axial length, as such a duct could cause small lesions in the ureter 2 and would also be decidedly redundant for some of the uses to which it needs to be put.

Nowadays there is a tendency to consider that a ureteral catheter stent is a means for facilitating the flow of urine rather than an outlet means for urine: ureteral peristalsis is thus maintained unaltered.

Ureteral catheter stents with smaller and/or variable diameters tend to maintain unaltered the trophic and peristaltic properties of the ureter.

It is intended that the invention herein described should not be limited to the forms of embodiment herein illustrated and described, which are to be considered as examples of embodiment of a ureteral catheter stent, which may undergo further modification in terms of the shape and arrangement or its parts or of details pertaining to its construction and assembly.

## Claims

1. A ureteral catheter stent (1) comprising a main duct (3) extending along a respective longitudinal axis (A), and two end portions (4) of the duct (3) which are substantially semi-circular, and which are characterised by the fact that the said main duct (3) comprises an intermediate linking portion (13) between the said two end portions (4) presenting a diameter ⌀ᵢ which is variable along the said longitudinal axis (A).

2. Ureteral catheter stent according to Claim 1, characterised by the fact that the said main duct (3) comprises two tubular portions (11, 12) interposed between one relative end portion (4) and the said intermediate portion (13), each tubular portion (11, 12) presenting a respective diameter (⌀₁, ⌀₂) which is constant along the said longitudinal axis (A).

3. Ureteral catheter stent according to Claim 1 or Claim 2, characterised by the fact that a first end portion (4) is a proximal portion, and the relative tubular portion (11) (12) presents a diameter (⌀₁) (⌀₂) which is greater than the diameter (⌀₂) (⌀₁) of the other tubular portion (12) (11).

4. Ureteral catheter stent according to Claim 1 or Claim 2, characterised by the fact that a first end portion (4) is a distal portion, and that the relevant tubular portion (12) (11) presents a diameter (⌀₁) (⌀₂) which is less than the diameter (⌀₂) (⌀₁) of the other tubular portion (11) (12).

5. Ureteral catheter stent according to Claim 2, Claim 3 or Claim 4, characterised by the fact that it comprises a number of holes (14) for lubrication obtained along the said duct (3).

6. Ureteral catheter stent according to Claim 5, characterised by the fact that the said holes (14) for lubrication are distributed along at least one tubular portion (11) (12) of the said two tubular portions (11, 12).

7. Ureteral catheter stent according to Claim 6, characterised by the fact that the said holes (14) for lubrication are distributed along the said two tubular portions (11, 12).

8. Ureteral catheter stent according to Claim 6 or Claim 7, characterised by the fact that the said holes (14) for lubrication are distributed along the said intermediate linking portion (13).
